# EUROPEAN PATENT APPLICATION

(11) **EP 4 382 106 A1**
(43) Date of publication of application: **12.06.2024**
(21) Application number: 22788373.3
(22) Date of filing: 11.04.2022
(51) Int. Cl.: A61K 31/541, A61K 35/28, A61P 29/00, C12N 5/0775, C12P 17/14

(54) **COMPOSITION FOR INHIBITING INFLAMMATION OR AGING OF STEM CELLS, AND METHOD FOR INHIBITING INFLAMMATION OR AGING BY USING SAME**

(30) Priority: 12.04.2021 KR 20210047171
(71) Applicant: MitoImmune Therapeutics Inc., Seoul 06123 (KR)
(72) Inventor: KIM, Soon Ha, Seoul 06123 (KR); LEE, Jae Ho, Seoul 06123 (KR); YU, Won Dong, Seoul 06123 (KR)
(74) Representative: Plasseraud IP
(86) International application number: PCT/KR2022/005208
(87) International publication number: WO 2022/220517

(57) **Abstract**

The present invention relates to a composition for inhibiting the inflammation or aging of stem cells, comprising, as an active ingredient, a compound of Chemical Formula 1 or a pharmaceutically acceptable salt thereof; a method for inhibiting the inflammation or aging of stem cells by using the same; and a method for preparing stem cells having inhibited inflammation or aging; and a pharmaceutical composition for preventing or treating inflammatory diseases, comprising the stem cells, wherein when stem cells are treated with the compound of Chemical Formula 1, the inflammation or aging of stem cells can be inhibited so that the function and morphology of stem cells and intracellular mitochondria damaged in an inflammatory environment can be restored, and the stem cells treated with the compound of Chemical Formula 1 can be effectively used in the prevention or treatment of inflammatory diseases.

## Description

### [Technical Field]

The present invention relates to a composition for inhibiting the inflammation or aging of stem cells, comprising, as an active ingredient, a compound of Chemical Formula 1 or a pharmaceutically acceptable salt thereof, and a method for inhibiting the inflammation or aging of stem cells.

### [Background Art]

As stem cells are capable of self-renewal and have the ability to differentiate into various cells, they are useful as cell therapeutic agents in fields such as regenerative medicine and tissue engineering, and recently, their usefulness and range of application have expanded to plastic surgery and cosmetology.

In particular, mesenchymal stem cells (MSCs) have multipotency to differentiate into adipocytes, osteocytes and chondrocytes. Further, mesenchymal stem cells are used in stem cell therapy due to their anti-inflammatory and tissue regeneration abilities.

Mesenchymal stem cells exhibit a unique metabolic function called quiescence, which exhibits a low level of metabolism that relies mainly on glycolysis, rather than mitochondria. Recently, it has been revealed that energy production by mitochondria in the metabolic process of mesenchymal stem cells is important for the proliferation, differentiation, and survival of stem cells, and the role of mitochondria in maintaining sternness is attracting attention. Therefore, maintaining the mitochondrial function of mesenchymal stem cells is becoming increasingly important to ensure their availability for stem cell therapy as well as tissue regeneration and homeostasis.

However, the characteristics of stem cells in MSCs may be changed by many factors such as DNA damage, mitochondrial dysfunction, and accumulation of toxic metabolites *in vivo* and *ex vivo.* The main causes of the outcome described above include age-related chronic damage and the like including pro-inflammatory cytokines as the immune system is changing.

For example, mesenchymal stem cells may be aged by exposure to pro-inflammatory cytokines such as TNF-α or IFN-γ, and damage to mesenchymal stem cells may occur, such as mitochondrial dysfunction and abnormalities of mitochondrial homeostasis.

It is known that sternness is lost when stem cells are damaged as described above, and the disturbance of the immune system and an imbalance in homeostasis caused by this may be directly or indirectly involved in the progression of chronic inflammatory diseases and aging.

Therefore, it is important to restore damaged sternness to a normal level. However, the understanding of the damaged cytoskeletal structure and phenotype of stem cells in an inflammatory environment is currently insufficient, and the development of a method for restoring the damaged sternness to a normal level or inhibiting the occurrence of the inflammation or aging of stem cells is not up to expectations.

### [Related Art Document]

### [Patent Document]

International Publication No. WO2009/025478

### [Disclosure]

### [Technical Problem]

The present invention has been made in an effort to provide a material for recovering stem cells from damage as described above, inhibiting the inflammation of stem cells not only *ex vivo* but also *in vivo,* and inhibiting aging.

Thus, an object of the present invention is to provide a composition for inhibiting the inflammation or aging of stem cells, comprising, as an active ingredient, a compound of Chemical Formula 1 or a pharmaceutically acceptable salt thereof, and a method for inhibiting the inflammation or aging of stem cells using the ingredient.

### [Technical Solution]

The present inventors confirmed that, when stem cells are exposed to an inflammatory environment, for example, inflammatory cytokines, a compound of Chemical Formula 1 can protect the stem cells and restore damaged sternness, thereby completing the present invention.

Thus, the present invention provides a composition for inhibiting the inflammation or aging of stem cells, comprising, as an active ingredient, a compound of Chemical Formula 1 or a pharmaceutically acceptable salt thereof.

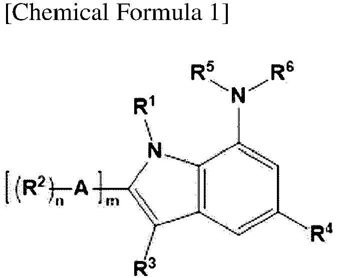

The present invention also provides a method for inhibiting the inflammation or aging of stem cells, the method comprising: treating stem cells with a compound of Chemical Formula 1 or a pharmaceutically acceptable salt thereof.

The present invention also provides a method for preparing inflammation- or aging-inhibited stem cells, the method comprising: treating stem cells with a compound of Chemical Formula 1 or a pharmaceutically acceptable salt thereof, and stem cells produced by a method for producing the same.

In addition, the present invention provides a pharmaceutical composition for preventing or treating inflammatory diseases, comprising stem cells produced by the production method as an active ingredient.

### [Advantageous Effects]

According to the composition or method according to the present invention, the inflammation or aging of stem cells can be inhibited when stem cells are treated with the compound of Chemical Formula 1. The compound of Chemical Formula 1 can restore the characteristics of stem cells damaged in an inflammatory environment, and specifically, it can restore mitochondrial quiescence in stem cells or improve homeostasis, regulate the cell cycle of stem cells, maintain sternness, and restore the phenotype of stem cells damaged in an inflammatory environment to a normal level. In addition, the compound of Chemical Formula 1 can restores the function, dynamics, morphology, and the like of mitochondria, and can be usefully used for mitochondria-associated inflammatory diseases.

Furthermore, inflammation- or aging-inhibited stem cells by the composition and method according to the present invention can be usefully used for the prevention or treatment of inflammatory diseases.

### [Description of Drawings]

FIG. 1A illustrates a set of bright field images and a set of fluorescence images according to Experimental Example 1 of the present invention.
FIG. 1B illustrates the expression level of NANOG, a sternness gene, according to Experimental Example 1 of the present invention.
FIG. 1C illustrates the expression level of p16, a cell cycle-related gene, according to Experimental Example 1 of the present invention.
FIG. 1D illustrates a graph confirming the cell viability at each concentration according to Experimental Example 1 of the present invention.
FIG. 2A illustrates mitochondrial membrane potential (red) and mitochondrial distribution (green) according to Experimental Example 2 of the present invention by confocal microscopic analysis.
FIG. 2B illustrates a graph of relative expression levels of mitochondrial dynamics-related genes according to Experimental Example 2 of the present invention.
FIG. 3A illustrates the oxygen consumption rate (OCR) according to Experimental Example 3 of the present invention.
FIG. 3B illustrates a basal respiration comparison graph according to Experimental Example 3 of the present invention.
FIG. 3C illustrates an ATP production comparison graph according to Experimental Example 3 of the present invention.
FIG. 3D illustrates a proton leakage comparison graph according comparison graph to Experimental Example 3 of the present invention.
FIG. 3E illustrates a spare respiratory capacity (SRC) comparison graph according to Experimental Example 3 of the present invention.
FIG. 3F illustrates a ROS assay graph according to Experimental Example 3 of the present invention.
FIG. 4A illustrates IL-1β expression levels according to Experimental Example 4 of the present invention.
FIG. 4B illustrates IL-6 expression levels according to Experimental Example 4 of the present invention.
FIG. 4C illustrates MCP-1 expression levels according to Experimental Example 4 of the present invention.
FIG. 4D illustrates western blotting analysis results and phosphorylation-induced activation ratios of AKT/mTOR/S6K according to Experimental Example 4 of the present invention.

### [Modes of the Invention]

Hereinafter, the present invention will be described in detail.

Meanwhile, each description and embodiment disclosed in the present application may also be applied to other descriptions and embodiments. That is, all combinations of various elements disclosed in the present application fall within the scope of the present invention. Further, the scope of the present application cannot be said to be limited by the specific description described below.

When a part "comprises" a component, it means that it can further include other components, rather than excluding other components, unless specifically stated otherwise. The present invention provides a composition for inhibiting the inflammation or aging of stem cells, comprising, as an active ingredient, a compound of Chemical Formula 1 or a pharmaceutically acceptable salt thereof. 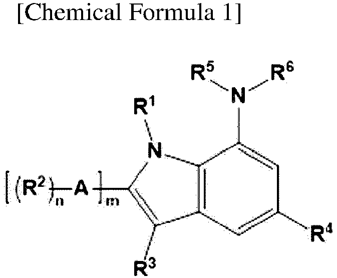 wherein,
n is an integer from 1 to 3,
m is 0 or 1,
A represents phenyl,
R¹ is hydrogen, or a C₁-C₆ alkyl,
R² represents hydrogen, a halogen or a C₁-C₆ alkoxy, or represents hydroxy-C₁₋C₆ alkyl, -(CH₂)ₚCO₂R⁷, -NHR⁸, -N(H)S(O)₂R⁷ or -NHC(O)R⁷, wherein p is an integer from 0 to 3, R⁷ represents hydrogen or a C₁-C₃ alkyl, and R⁸ represents (C₁₋C₃ alkyl)piperidinyl, or (C₁₋C₃ alkyl)sulfonyl,
R³ represents hydrogen, a halogen, a C₁-C₆ alkyl or phenyl, or represents - (CH₂)ₚ-heterocycle in which the heterocycle is a 5- to 6-membered ring containing one or two heteroatom(s) selected among N and O atoms, wherein p is an integer from 0 to 3, provided that R³ is phenyl when m is 0,
R⁴ represents a halogen, a C₁-C₆ alkyl, hydroxy-C₁₋C₆ alkyl, -O-phenyl, - (CH₂)ₚCO₂R⁷, -(CH₂)ₚ-heterocycle in which the heterocycle is a 5- to 6-membered ring containing one or two heteroatom(s) selected among N and O atoms, or proline-N-carbonyl, wherein p is an integer from 0 to 3, and R⁷ is as described above,
R⁵ is hydrogen, or a C₁-C₆ alkyl, and
R⁶ represents a C₁-C₆ alkyl, a C₃-C₆ cycloalkyl, a heterocycle or heterocyclyl-C₁₋C₆ alkyl, wherein the heterocycle is a 3- to 8-membered ring including 1 to 3 heteroatom(s) selected among N and O atoms, and R⁶ may be substituted with (C₁-C₆ alkyl)amine, hydroxy-C₁₋C₆ alkyl, or (C₁-C₆ alkyl)sulfonyl. The compound of Chemical Formula 1 is a compound disclosed by International Publication No. WO2009-025478, and is a material known to exhibit preventive or therapeutic and ameliorative effects on cell necrosis and related diseases.

In the present invention, stem cells damaged in an inflammatory environment were treated with the compound of Chemical Formula 1 to restore stem cells by the compound of Chemical Formula 1, and since the effect of inhibiting inflammation or aging was confirmed, the present invention identified a novel use of the compound of Chemical Formula 1.

The compound of Chemical Formula 1 of the present invention may be used in the form of a pharmaceutically acceptable salt thereof. In particular, the pharmaceutically acceptable salt may be an acid addition salt formed by a free acid. Here, the acid addition salt may be obtained from an inorganic acid such as hydrochloric acid, nitric acid, phosphoric acid, sulfuric acid, hydrobromic acid, hydroiodic acid, nitrous acid, and phosphorous acid, a non-toxic organic acid such as aliphatic mono and dicarboxylates, phenyl-substituted alkanoates, hydroxy alkanoates and alkanedionates, aromatic acids, and aliphatic and aromatic sulfonic acid, and an organic acid such as trifluoroacetic acid, acetate, benzoic acid, citric acid, lactic acid, maleic acid, gluconic acid, methanesulfonic acid, 4-toluenesulfonic acid, tartaric acid, and fumaric acid. Types of such pharmaceutically acceptable salts may include sulfates, pyrosulfates, bisulfates, sulfites, bisulfites, nitrites, phosphates, monohydrogen phosphates, dihydrogen phosphates, metaphosphates, pyrophosphate chlorides, bromides, iodides, fluorides, acetates, propionates, and the like

The composition of the present invention may include not only the compound of Chemical Formula 1 or a pharmaceutically acceptable salt thereof, but also all salts, isomers, hydrates and/or solvates thereof that may be prepared by typical methods.

As used herein, the "isomer" may refer to a compound of the invention, which has the same chemical or molecular formula but is structurally or sterically different, or a salt thereof. Such isomers include all of a structural isomer such as a tautomer, an R or S isomer having an asymmetric carbon center, an isomer such as a geometric isomer (trans, cis), and an optical isomer (enantiomer). All of these isomers and mixtures thereof are also included within the scope of the present invention.

As used herein, the "hydrate" may refer to a compound of the present invention including a stoichiometric or non-stoichiometric amount of water bonded by a non-covalent intermolecular force, or a salt thereof. The hydrate of the compound represented by Chemical Formula 1 of the present invention may include a stoichiometric or non-stoichiometric amount of water bonded by a non-covalent intermolecular force. The hydrate may contain at least 1 equivalent, preferably 1 to 5 equivalents of water. Such a hydrate may be prepared by crystallizing the compound represented by Chemical Formula 1 of the present invention, an isomer thereof, or pharmaceutically acceptable salt thereof from water or a solvent containing water.

As used herein, the "solvate" may refer to a compound of the present invention including a stoichiometric or non-stoichiometric amount of solvent bonded by a non-covalent intermolecular force, or a salt thereof. Preferred solvents in this regard include volatile, non-toxic, and/or solvents suitable for administration to humans.

As used herein, the term 'alkyl' refers to an aliphatic hydrocarbon radical. The alkyl may be either a "saturated alkyl" containing no alkenyl or alkynyl moiety or an "unsaturated alkyl" containing at least one alkenyl or alkynyl moiety, and may have 1 to 20 carbon atoms, unless otherwise defined.

The term 'alkoxy' refers to an alkyl-oxy having 1 to 10 carbon atoms, unless otherwise defined.

The term 'cycloalkyl' refers to a saturated aliphatic 3- to 10-membered ring, unless otherwise defined. Typical cycloalkyl groups include cyclopropyl, cyclobutyl, cyclopentyl, cyclohexyl, and the like, but are not limited thereto.

Unless otherwise defined, the term 'heterocycle' refers to a 3- to 10-membered ring, which includes 1 to 3 heteroatoms selected from the group consisting of N, O and S, may be fused with benzo or a C₃-C₈ cycloalkyl, and is saturated or includes one or two double bond(s), preferably a 4- to 8-membered ring, and more preferably a 5- to 6-membered ring. Further, the term may be used interchangeably with the term 'heterocyclyl.' Examples of the heterocycle include pyrroline, pyrrolidine, imidazoline, imidazolidine, pyrazolidine, pyran, piperidine, morpholine, thiomorpholine, piperazine, hydrofuran, and the like, but are not limited thereto.

All other terms and abbreviations used herein, unless otherwise defined, are to be interpreted as commonly understood by a person with ordinary skill in the art to which this invention pertains.

In an exemplary embodiment of the present invention, in the compound of Chemical Formula 1,
R³ represents hydrogen, a halogen, or phenyl, or represents -(CH₂)ₚ-heterocycle in which the heterocycle is morpholino or piperazinonyl, wherein p is an integer from 0 to 1, provided that R³ may be phenyl when m is 0.

In an exemplary embodiment of the present invention, in the compound of Chemical Formula 1,
R⁴ is a halogen, a C₁-C₃ alkyl, hydroxy-C₁₋C₃ alkyl, -O-phenyl, -(CH₂)ₚCO₂-ethyl, -(CH₂)ₚ-heterocycle in which the heterocycle is thiomorpholino, morpholino, piperazinonyl, or pyrrolidinyl, or proline-N-carbonyl, wherein p may be an integer from 0 to 1.

In an exemplary embodiment of the present invention, in the compound of Chemical Formula 1,
R⁵ is hydrogen, or a C₁-C₃ alkyl, and
R⁶ represents a C₁-C₃ alkyl, a C₃-C₆ cycloalkyl, a heterocycle or heterocyclyl-C₁-C₃ alkyl, wherein the heterocycle may be tetrahydro-2H-pyran, or piperidinyl, and when R⁶ is a heterocycle or heterocyclyl-C₁-C₃ alkyl, R⁶ may be substituted with (C₁-C₆ alkyl)amine, hydroxy-C₁₋C₆ alkyl, or (C₁-C₆ alkyl)sulfonyl.

In the present invention, examples of the compound of Chemical Formula 1 include Compounds 1 to 32 listed in the following Table 1, or pharmaceutically acceptable salts thereof.

**[Table 1]**

| Cmpd # | Structure | Chemical name |
|---|---|---|
| 1 | 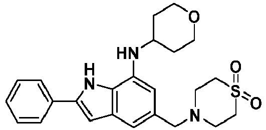 | 5-[(1,1-dioxido-4-thiomorpholinyl)methyl]-2-phenyl-N-(tetrahydro-2H-pyran-4-yl)-1H-indole-7-amine |
| 2 | 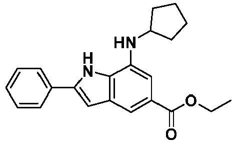 | ethyl 7-(cyclopentylamino)-2-phenyl-1H-indole-5-carboxylate |
| 3 | 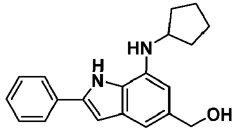 | (7-(cyclopentylamino)-2-phenyl-1H-indole-5-yl)methanol |
| 4 | 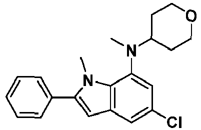 | 5-chloro-N, 1-dimethyl-2-phenyl-N-(tetrahydro-2H-pyran-4-yl)-1H-indole-7-amine |
| 5 | 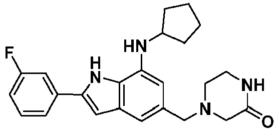 | 4-((7-(cyclopentylamino)-2-(3-fluorophenyl)-1H-indol-5-yl)methyl)piperazine-2-one |
| 6 | 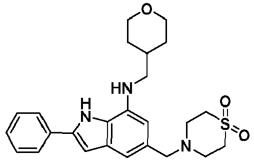 | 4-((2-phenyl-7-(((tetrahydro-2H-pyran-4-yl)methyl)amino)-1H-indol-5-yl)methyl)thiomorpholine 1,1-dioxide |
| 7 | 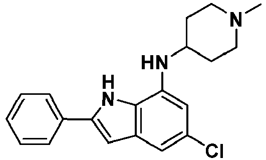 | 5-chloro-N-(1-methylpiperidin-4-yl)-2-phenyl-1H-indole-7-amine |
| 8 | 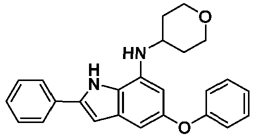 | 5-phenoxy-2-phenyl-N-(tetrahydro-2H-pyran-4-yl)-1H-indole-7-amine |
| 9 | 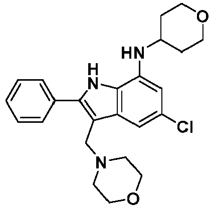 | 5-chloro-3-(morpholinomethyl)-2-phenyl-N-(tetrahydro-2H-pyran-4-yl)-1H-indole-7 -amine |
| 10 | 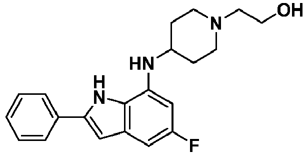 | 2-(4-((5-fluoro-2-phenyl-1H-indol-7-yl)amino)piperidin-1-yl)ethan-1-ol |
| 11 | 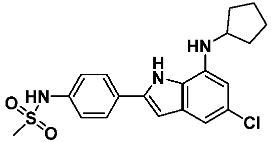 | N-(4-(5-chloro-7-(cyclopentylamino)-1H-indole-2-yl)phenyl)methanesulfonamide |
| 12 | 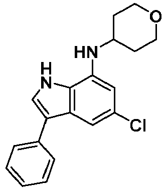 | 5-chloro-3-phenyl-N-(tetrahydro-2H-pyran-4-yl)-1H-indole-7-amine |
| 13 | 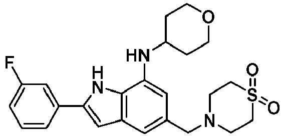 | 4-((2-(3-fluorophenyl)-7-((tetrahydro-2H-pyran-4-yl)amino)-1H-indol-5-yl)methyl)thiomorphline 1,1-dioxide |
| 14 | 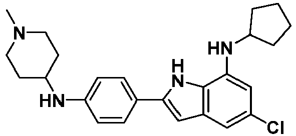 | 5-chloro-N-cyclopentyl-2-(4-((1-methylpiperidin-4-yl)amino)phenyl)-1H-indole-7-amine |
| 15 | 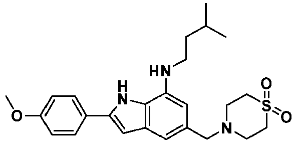 | 4-((7-(isopentylamino)-2-(4-methoxyphenyl)-1H-indol-5-yl)methyl)thiomorpholine 1,1-dioxide |
| 16 | 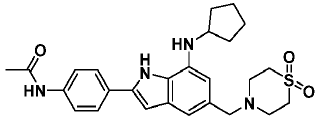 | N-(4-(7-(cyclopentylamino)-5-((1,1-dioxidothiomorpholino)methyl)-1H-indole-2-yl)phenyl)acetamide |
| 17 | 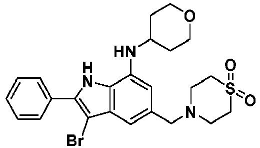 | 4-((3-bromo-2-phenyl-7-((tetrahydro-2H-pyran-4-yl)amino)-1H-indole-5-yl)methyl)thiomorpholine 1,1-dioxide |
| 18 | 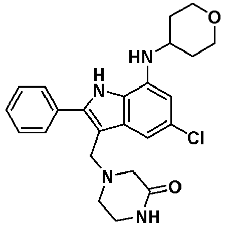 | 4-((5-chloro-2-phenyl-7-((tetrahydro-2H-pyran-4-yl)amino)-1H-indol-3-yl)methyl)piperazine-2-one |
| 19 | 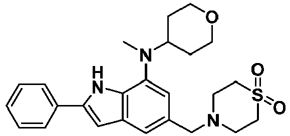 | 4-((7-(methyl(tetrahydro-2H-pyran-4-yl)amino)-2-phenyl-1H-indol-5-yl)methyl)thiomorpholine 1,1-dioxide |
| 20 | 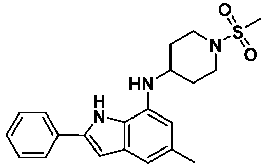 | 5-methyl-N-(1-(methylsulfonyl)piperidin-4-yl)-2-phenyl- 1H-indole-7-amine |
| 21 | 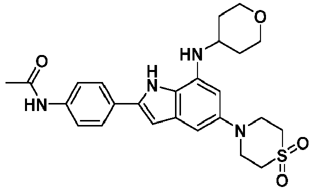 | N-(4-(5-(1,1-dioxidothiomorpholino)-7-((tetrahydro-2H-pyran-4-yl)amino)-1H-indol-2-yl)phenyl)acetamide |
| 22 | 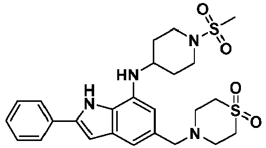 | 4-((7-((1-(methylsulfonyl)piperidin-4-yl)amino)-2-phenyl-1H-indole-5-yl)methyl)thiomorpholine 1,1-dioxide |
| 23 | 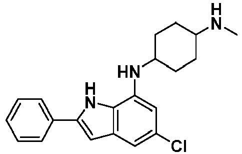 | N1-(5-chloro-2-phenyl-1H-indol-7-yl)-N4-methylcyclohexane-1,4-diamine |
| 24 | 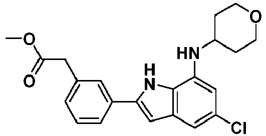 | methyl 2-(3-(5-chloro-7-((tetrahydro-2H-pyran-4-yl)amino)-1H-indol-2-yl)phenyl)acetate |
| 25 | 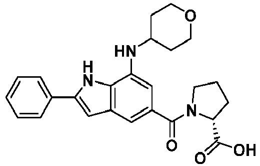 | (2-phenyl-7-((tetrahydro-2H-pyran-4-yl)amino)-1H-indol-5-carbonyl)-D-proline |
| 26 | 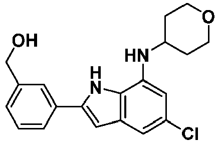 | (3-(5-chloro-7-((tetrahydro-2H-pyran-4-yl)amino)-1H-indol-2-yl)phenyl)methanol |
| 27 | 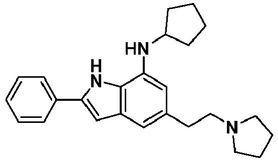 | N-cyclopentyl-2-phenyl-5-(2-(pyrrolidin-1-yl)ethyl)-1H-indole-7-amine |
| 28 | 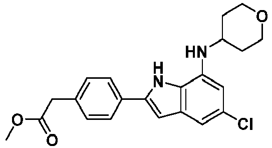 | methyl 2-(4-(5-chloro-7-((tetrahydro-2H-pyran-4-yl)amino)-1H-indol-2-yl)phenyl)acetate |
| 29 | 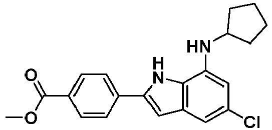 | methyl 4-(5-chloro-7-(cyclopentylamino)-1H-indol-2-yl)benzoate |
| 30 | 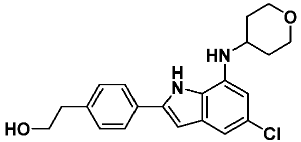 | 2-(4-(5-chloro-7-((tetrahydro-2H-pyran-4-yl)amino)-1H-indol-2-yl)phenyl)ethan-1-ol |
| 31 | 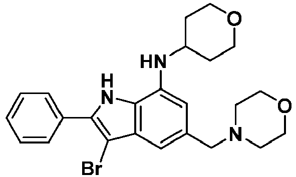 | 3-bromo-5-(morpholinomethyl)-2-phenyl-N-(tetrahydro-2H-pyran-4-yl)-1H-indole-7-amine |
| 32 | 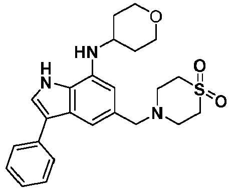 | 4-((3-phenyl-7-((tetrahydro-2H-pyran-4-yl)amino)-1H-indol-5-yl)methyl)thiomorpholine 1,1-dioxide |

In preferable exemplary embodiments of the present invention, the compound of Chemical Formula 1 may be a compound of the following Chemical Formula 2.

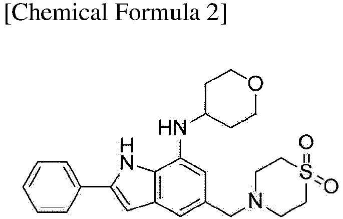

In the present invention, stem cells may include induced pluripotent stem cells, embryonic stem cells, adult stem cells, and may specifically be adult stem cells. The adult stem cells may be mesenchymal stem cells, human tissue-derived mesenchymal stromal cells, human tissue-derived mesenchymal stem cells, multipotent stem cells, or amniotic epithelial cells, but are not limited thereto.

The stem cells may be isolated from any one selected from the group consisting of bone marrow, an umbilical cord, umbilical cord blood, fat, muscle, skin, an amniotic membrane, and a placenta.

In the present invention, the stem cells may be mesenchymal stem cells, mesenchymal stem cells derived from various adult tissues such as fat, bone marrow, umbilical cord blood, a placenta, muscle, and nerve tissue may be used as the mesenchymal stem cells, and according to an exemplary embodiment, the mesenchymal stem cells may be mesenchymal stem cells derived from the placenta, but are not limited thereto.

In the present specification, the stem cells may be *in vivo* or *ex vivo* stem cells. In the case of *ex vivo* stem cells, they may be stem cells cultured in a medium, and any common medium used in the art suitable for cell culture may be used as the medium.

As used herein, the term "inhibition of inflammation" means that, when cells are exposed in an inflammatory environment, they can overcome the inflammatory environment to exhibit the same morphology as normal cells, and the inflammatory environment may artificially expose inflammatory cytokines, but the inflammatory cytokines may also be expressed naturally in cells. In particular, the inhibition of inflammation may refer to restoring mitochondrial damage caused by inflammatory cytokines to normal levels.

As used herein, the term "cellular senescence" means that the natural aging phenomenon, in which the functions of biological organs decline over time, occurs at the cellular level, and means that for example, the growth and division of cells are suspended or significantly delayed by various physical, chemical, and biological stresses (for example, high oxygen concentration conditions during continuous subculture and *ex vivo* culture) that cells are subjected to either internally or externally. In undifferentiated cells such as stem cells, cellular senescence is a concept that includes the process in which stem cells lose their phenotype in an undifferentiated state and lose their multipotency or pluripotency.

In the present specification, the compound of Chemical Formula 1 or a pharmaceutically acceptable salt thereof in the composition may be included at a concentration of 0.05 µM to 10 µM. According to an exemplary embodiment, the concentration may be 0.1 µM to 5.0 µM, 0.2 µM to 4 µM, 0.5 µM to 4 µM, 0.5 µM to 3 µM, 0.8 µM to 3 µM, 0.8 µM to 2 µM, or 0.8 µM to 1.5 µM, but is not limited thereto. However, when the concentration is less than the above range, the effect may not be exhibited, and when the concentration exceeds the above range, cytotoxicity may be exhibited.

The concentration may be a concentration of the compound of Chemical Formula 1 in the medium in the case of *ex vivo* stem cells, and may be the concentration of the compound of Chemical Formula 1 in the composition administered to the body in the case of *in vivo* stem cells, but is not limited thereto.

In the present invention, the inhibition of inflammation or aging of stem cells may restore stem cells damaged by exposure to inflammatory cytokines to normal levels. Here, the normal level may refer to the level of stem cells that have not been exposed to an inflammatory environment such as inflammatory cytokines.

The exposure to inflammatory cytokines artificially or naturally provides stem cells with an inflammatory environment, and the present invention confirmed the inhibition of the inflammation or aging of stem cells by treating stem cells in such an inflammatory environment with the compound of Chemical Formula 1.

The inflammatory cytokine may be one or more selected from the group consisting of IL-1α, IL-1β, IL-18, IL-6, IL-8, IL-17, TNF-α and IFN-γ and may be more specifically TNF-α and/or IFN-γ, but is not limited thereto.

Such inflammatory cytokines may hyperactivate mitochondrial dynamics and metabolism and thus increase ROS levels, SASP production, morphological heterogeneity, irregular cytoskeletal structures, and more.

The present inventors confirmed that ROS levels, SASP production, morphological heterogeneity, irregular cytoskeletal structures, and the like are restored to normal levels in stem cells damaged by inflammatory cytokines as described above, and confirmed the inhibition of inflammation or aging of stem cells.

Accordingly, the inhibition of inflammation or aging of stem cells may have one or more characteristics selected from the group consisting of restoration of quiescence of stem cells or improvement of homeostasis; cell cycle regulation or maintenance of sternness; and restoration of the phenotype of stem cells.

In particular, although it was confirmed that the exposure to inflammatory cytokines increases the expression of a senescence-associated secretory phenotype (SASP), it was confirmed that cellular senescence was inhibited because the expression of SASP was significantly reduced in the case of stem cells treated with the compound of Chemical Formula 1.

The inhibition of inflammation or aging characteristics of stem cells may be for stem cells exposed to an inflammatory environment.

"Quiescence" is a unique state of stem cells, and is a dormant state with minimal basal activity. It is important for stem cells to maintain quiescence, and according to the present invention, although it can be seen that quiescence may be abnormally switched to an active state in an inflammatory environment, quiescence can be maintained due to treatment with the compound of Chemical Formula 1.

"Sternness" means that stem cells have the ability to survive in tissues for a long time and self-renew, and have the ability to recreate a required cell group through the process of differentiation again under circumstances such as tissue damage. That is, it refers to sternness as a concept encompassing the characteristics of self-renewal and multipotency.

The compound of Chemical Formula 1 of the present invention or a pharmaceutically acceptable salt thereof may restore sternness damaged in an inflammatory environment, and may enhance sternness so as to have resistance to an inflammatory environment.

The present invention also provides a method for inhibiting the inflammation or aging of stem cells, the method comprising: treating stem cells with a compound of Chemical Formula 1 or a pharmaceutically acceptable salt thereof.

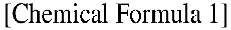

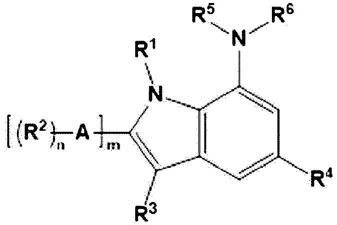

In the formula, R¹ to R⁶, A, n and m are each the same as defined above.

The present invention also provides a method for preparing inflammation- or aging-inhibited stem cells, the method comprising: treating stem cells with a compound of Chemical Formula 1.

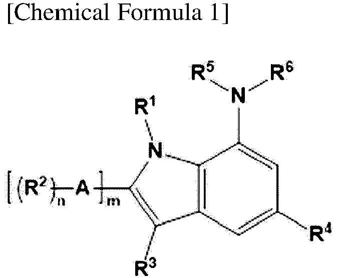

In the formula, R¹ to R⁶, A, n and m are each the same as defined above.

In the treating of the stem cells with the compound of Chemical Formula 1 or the pharmaceutically acceptable salt thereof, the stem cells may be stem cells in which inflammatory activity is induced. The induction of inflammatory activity may mean that stem cells are cultured in an inflammatory environment, and the inflammatory environment may expose stem cells to inflammatory cytokines.

The treating of the stem cells with the compound of Chemical Formula 1 or the pharmaceutically acceptable salt thereof may be performed *ex vivo*, *in vitro*, or *in vivo.* Alternatively, the treatment step may mean that the compound of Chemical Formula 1 or the pharmaceutically acceptable salt thereof is added to stem cells cultured in a medium, or that the compound of Chemical Formula 1 or the pharmaceutically acceptable salt thereof is also administered *in vivo.*

By enabling stem cells to inhibit inflammation or aging in an inflammatory environment, stem cells with stem cell characteristics suitable as cell therapeutic agents may be provided.

Accordingly, the present invention provides stem cells with inhibited inflammation or aging prepared by the method for preparing stem cells.

In addition, since it was confirmed that the morphological improvement of the cytoskeletal structure and the restoration of phenotype are possible upon treatment with the compound of Chemical Formula 1, the present invention provides a composition for restoring stem cells damaged by an inflammatory environment, comprising the compound of Chemical Formula 1 or a pharmaceutically acceptable salt thereof.

For the restoration of damaged stem cells, the compound of Chemical Formula 1 is capable of restoring the distribution or morphology of mitochondria damaged by the inflammatory environment, reducing mitochondrial oxidative stress, improving mitochondrial quiescence and homeostasis, and restoring the phenotype of damaged stem cells.

Accordingly, the compound of Chemical Formula 1 may also be used in stem cell therapy to prevent or treat mitochondria-associated inflammatory diseases.

Furthermore, the present invention provides a pharmaceutical composition for preventing or treating inflammatory diseases, comprising the inflammation- or aging-inhibited stem cells prepared according to the present invention as an active ingredient.

Stem cells treated with the compound of Chemical Formula 1 of the present invention, or a pharmaceutically acceptable salt thereof may be included as an active ingredient of a cell therapy agent by activating stem cells in a state suitable for transplantation and modifying the characteristics thereof to suit the human body.

The inflammatory disease may be selected from the group consisting of dermatitis, allergies, atopy, asthma, conjunctivitis, periodontitis, rhinitis, tympanitis, pharyngolaryngitis, tonsillitis, pneumonia, gastric ulcer, gastritis, Crohn's disease, colitis, peritonitis, osteomyelitis, phlegmon, cerebromeningitis, encephalitis, pancreatitis, stroke, acute bronchitis, chronic bronchitis, hemorrhoids, gout, ankylosing spondylitis, rheumatic fever, systemic lupus erythematosus, fibromyalgia, psoriatic arthritis, osteoarthritis, rheumatoid arthritis, infectious arthritis, periarthritis, tendinitis, tenosynovitis, peritendinitis, myositis, hepatitis, cystitis, nephritis, Sjogren's syndrome, multiple sclerosis, inflammatory bowel disease, transplantation rejection, bronchopulmonary dysplasia (BPD), chronic obstructive pulmonary disease (COPD) and acute and chronic inflammatory diseases.

As used herein, treatment refers to stopping or delaying the progress of the disease when used for a subject who shows the symptoms of the onset of the disease, and prevention refers to stopping or delaying the symptom of the onset of the disease when used for a subject that does not show, but is at risk of, the symptoms of the onset of the disease.

In the present invention, the "pharmaceutical composition" may contain a pharmaceutically acceptable carrier together with the compound of the present invention, if necessary.

The compound of Chemical Formula 1 according to the present invention may be administered in various oral and parenteral dosage forms, and during formulation, the compound of Chemical Formula 1 according to the present invention is prepared using a diluent or an excipient, such as a filler, a thickener, a binder, a wetting agent, a disintegrant, and a surfactant, which are commonly used.

A solid formulation for oral administration includes a tablet, a pill, a powder, a granule, a capsule, a troche, and the like, and the solid formulation is prepared by mixing at least one excipient, for example, starch, calcium carbonate, sucrose or lactose, gelatin, and the like with one or more compounds of the present invention. Further, in addition to a simple excipient, lubricants such as magnesium stearate and talc are also used. A liquid preparation for oral administration corresponds to a suspension, a liquid for internal use, an emulsion, a syrup, and the like, and the liquid preparation may include various excipients, for example, a wetting agent, a sweetener, an aromatic, a preservative, and the like, in addition to water and liquid paraffin, which are commonly used simple diluents.

A preparation for parenteral administration includes an aqueous sterile solution, a non-aqueous solvent, a suspension solvent, an emulsion, a freeze-dried preparation, a suppository, or the like. As a non-aqueous solvent and a suspension solvent, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurinum, glycerol, gelatin, and the like.

In addition, the effective dosage of the compound of Chemical Formula 1 of the present invention for the human body may vary depending on the patient's age, body weight, sex, administration form, health condition, and severity of disease, and is generally about 0.001 to 100 mg/kg/day, preferably 0.01 to 35 mg/kg/day. Based on an adult patient weighing 70 kg, the dosage is generally 0.07 to 7000 mg/day, preferably 0.7 to 2500 mg/day, and the compound of the present invention may be administered once or in several divided doses a day at regular time intervals according to the judgment of a doctor or pharmacist.

Further, the present invention provides a method for preventing or treating an inflammatory disease, the method comprising administering stem cells prepared by the preparation method to a subject in need.

In addition, the present invention provides a use of stem cells prepared by the preparation method for preventing or treating an inflammatory disease. The preventive or treatment method and use may apply to the same type of inflammatory disease as described above for the pharmaceutical composition.

As used herein, the term "subject in need" refers to a vertebrate such as a mammal including a human and a domesticated animal, and a bird, in which the inflammatory disease can be ameliorated, prevented or treated by administering the pharmaceutical composition according to the present invention.

As used herein, "administration" refers to the introduction of a predetermined material to a human or animal by any appropriate method, and for the route of administration of the therapeutic composition according to the present invention, the preventive or therapeutic composition according to the present invention may be orally or parenterally administered via any general route, which may reach a target tissue.

Numerical values set forth herein should be construed to include a range of equivalents, unless otherwise specified.

Hereinafter, the present invention will be described in detail with reference to the following Experimental Examples. However, the following Experimental Examples are only for exemplifying the present invention, and the content of the present invention is not limited by the following Experimental Examples. However, the Experimental Examples are provided merely for helping in understanding the present invention, and the scope of the present invention is not limited by the Examples in any sense.

### Preparation Example 1. Culture of mesenchymal stem cells and treatment with example compound

Human placenta-derived mesenchymal stem cells (hPD-MSCs) were harvested from inside the placental chorion, and the harvested cells were cultured in an alpha-Minimum Essential medium (Cytiva, Marlborough, MA, USA) supplemented with 10% fetal bovine serum (ThermoFisher, Waltham, MA, USA), 1% penicillin/streptomycin (ThermoFisher, Waltham, MA, USA), 25 ng/mL human fibroblast growth factor-4 (Peprotech, Rocky Hill, NJ, USA) and 1 µg/mL heparin (Merck, San Francisco, CA, USA). A total of 1 × 10⁵hPD-MSCs were seeded in a cell culture dish (90 × 20 mm) (SPL, Pocheon, Korea) and cultured at 37°C in an incubator containing 5% CO₂. The cells were subcultured every 4 days until reaching 70 to 80% confluency. Expanded cells were obtained at passage 12 or 13. During subculture, viable cells were stained with Trypan blue (Merck) and the number of cells was manually counted.

In a passage for sample extraction, hPD-MSCs were cultured for 24 hours in a medium with or without an example compound (5-[(1,1-dioxido-4-thiomorpholinyl)methyl]-2-phenyl-N-(tetrahydro-2H-pyran-4-yl)-1H-indole-7-amine) while including 2.5 ng/mL TNF-α (Peprotech) and 25 ng/mL IFN-γ (Peprotech).

According to Preparation Example 1, a "TNF-α/IFN-γ-exposed MSC control" (hereinafter, also referred to as "TI"), an "experimental group treated with the example compound in the TNF-α/IFN-γ exposed MSC control" (hereinafter, also referred to as "TIM"), and an "untreated MSC control not exposed to and untreated with both TNF-α/IFN-γ and the example compound (hereinafter, also referred to as "Control") were prepared.

### Experimental Example 1. Confirmation of improvement in morphology and restoration of sternness of TNF-α/IFN-γ-exposed MSCs according to treatment with example compound

### Experimental Example 1-1. Confirmation of MSC morphological image and fluorescence image of phalloidin-stained cells

Since the morphology of MSCs is a useful visual marker for the state of MSCs, the morphological restoration effect of the example compound was confirmed by the morphology of MSCs.

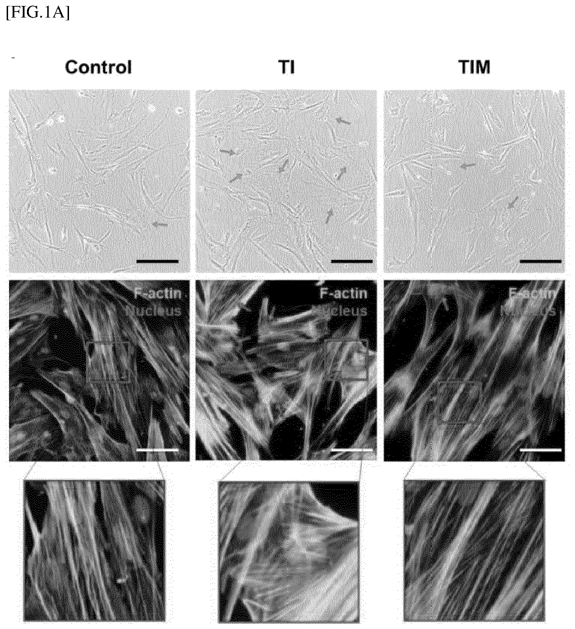

The morphology of MSCs was confirmed by bright-field images and fluorescence microscope images, stained cells were identified by phalloidin, which specifically labels the F-actin structure, and Hoechst 33342, which labels nuclei, in the fluorescence image, and the results are shown in FIG. 1A.

The upper panel of FIG. 1A shows a bright-field image, in which blue arrows indicate abnormal MSCs with thin and damaged spindles. The lower panel of FIG. 1A shows fluorescence microscope images, in which red arrows indicate portions in which the certain axial directions of intracellular microfilaments are disrupted, intersecting, or disorganized portions. In FIG. 1A, the black scale bar indicates 200 µm and the white scale bar indicates 100 µm.

As shown in Figure 1A, the MSC control untreated with TNF-α/IFN-γ and the example compound exhibited a thin and normal spindle shape, whereas the TNF-α/IFN-γ-exposed MSC control showed an increase in morphological heterogeneity, such as a flattened, elongated, and abnormal morphology.

In addition, as shown by the F-actin staining in FIG. 1A, it was confirmed that the untreated MSC control showed an organized F-actin structure with long parallel cell axes, whereas the TNF-α/IFN-γ exposed MSC control showed a collapsed cellular axis and a disorganized cytoskeletal structure.

However, it can be confirmed that, when the TNF-α/IFN-γ-exposed MSC control is treated with the example compound, abnormal morphology and disorganized microfilaments are remarkably improved to levels similar to those of the untreated MSC control. Therefore, it can be seen that the example compound improved the cytoskeletal structure and cell morphology of hPD-MSCs exposed to TNF-α/IFN-γ.

### Experimental Example 1-2. Evaluation of cell cycle and sternness

In the present experiment, the expression levels of stem cell differentiation-related genes and cell cycle-related genes were analyzed by qPCR.

Real-time qPCR was performed using an Easy-Spin Total RNA Kit (iNtRON, Seongnam, Korea) to extract total RNA from hPD-MSCs and RNA concentration was adjusted to 100 ng/µL using a microplate spectrophotometer (Epoch^{™} Microplate Spectrophotometer, BioTek, Winooski, VT, USA), and the extracted RNA was reverse transcribed into cDNA using RT PreMix (dT20) (Bioneer, Seoul, Korea) using a SimpliAmp Thermal Cycler (Life Technologies, Carlsbad, CA, USA). Real-time qPCR was performed using a real-time PCR machine (CFX Connect, Bio-Rad, Hercules, CA, USA), SYBR Green Supermix (Bio-Rad) and the relevant primers, and all experiments were performed in triplicate for statistical analysis. Primer sequences are shown in Table 2.

**[Table 2]**

| **Primer name** | **Gene code** | **Forward primer** | **PCR condition** | **Product size** |
|---|---|---|---|---|
| | | **Backward primer** | | |
| **hβ-actin** | X00351.1 | 5'-ACAATGTGGCCGAGGACTTT -3' | 58°C | 104 |
| | | 5'-TGTGTGGACTTGGGAGAGGA -3' | | |
| **hCCNA2** | NM_001237.5 | 5'-TGCTGACCCATACCTCAAGT-3' | 58°C | 211 |
| | | 5'-TG ACTGTTGTGCATGCTGTG-3 ' | | |
| **hP16** | NM_000077.4 | 5'- CGGAGGAAGAAAGAGGAGGG-3' | 58°C | 252 |
| | | 5'- GGCCTCCGACCGTAACTATT-3' | | |
| **hOCT4** | NM_002701.6 | 5'-GGAGTTTGTGCCAGGGTTTT-3' | 58°C | 238 |
| | | 5'-ACTTCACCTTCCCTCCAACC-3' | | |
| **hNANOG** | NM_024865.4 | 5'-AAGGTCCCGGTCAAGAAACA-3' | 58°C | 175 |
| | | 5'-TCTGCGTCACACCATTGCTA-3' | | |
| **hDRP1** | NM_001278464.1 | 5'- TCCATGAGACTTTTGGGCGA-3' | 58°C | 152 |
| | | 5'- TTGCCGCTTCACCAGTAACT-3' | | |
| **hMFN1** | NM_033540.3 | 5'- ACAAGGTGAATGAGCGGCTT-3' | 58°C | 136 |
| | | 5'- TCCACCAAGAAATGCAGGCA-3' | | |
| **hMFN2** | NM_014874.4 | 5'- ACCGTGATCAATGCCATGCT-3' | 58°C | 155 |
| | | 5'- TGCiTTCACAGTCTTGGCACT-3' | | |
| **hIL-1β** | NM_000576.3 | 5'- TCAGCACCTCTCAAGCAGAA-3' | 58°C | 136 |
| | | 5'- TCCACATTCAGCACAGGACT-3' | | |
| **hIL-6** | NM_000600.5 | 5'- AATAACCACCCCTGACCCAA-3' | 58°C | 153 |
| | | 5'- TGCTACATTTGCCGAAGAGC-3' | | |
| **hMCP-1** | NM_002982.4 | 5'- GCAAGTGTCCCAAAGAAGCT-3' | 58°C | 85 |
| | | 5'- TCCTGAACCCACTTCTGCTT-3' | | |

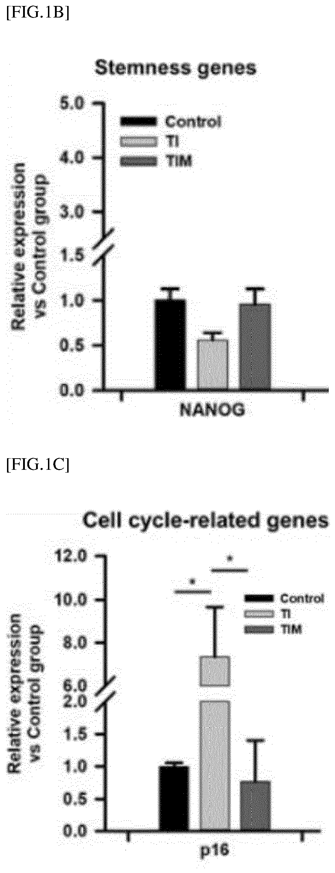

FIGS. 1B and 1C show the relative expression level compared to the untreated MSC control, specifically, FIG. 1B shows the expression level of NANOG, a stem cell differentiation potential gene, and FIG. 1C shows the expression level of p16 (cell cycle inhibitor), a cell cycle-related gene.

As shown in FIG. 1B, when the TNF-α/IFN-γ exposed MSC control (TI) was treated with the example compound, NANOG expression was significantly increased to a level similar to that of the untreated MSC control. Furthermore, as shown in FIG. 1C, exposure to TNF-α/IFN-γ increased p16, but treatment with the example compound remarkably decreased p16.

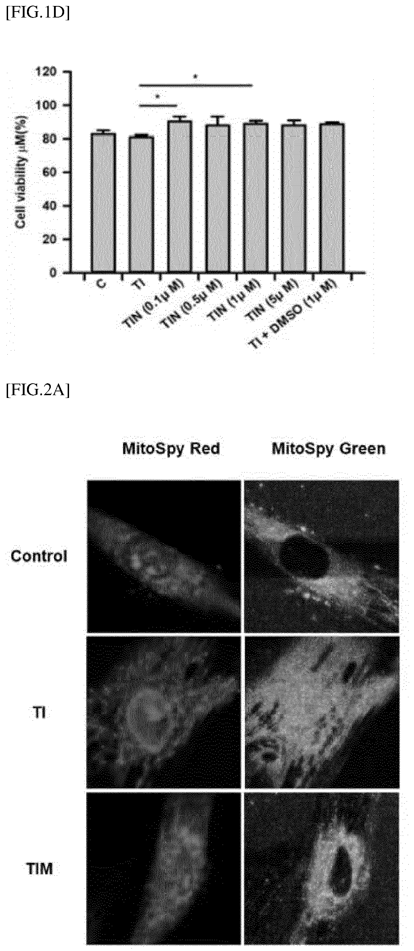

Meanwhile, upon exposure to TNF-α/IFN-γ and treatment with the example compound, cell viability was evaluated, and the results are shown in FIG. 1D. Although TNF-α and IFN-γ were reported to induce necrotic cell death in other cell types, as can be confirmed in FIG. 1D, cell viability was high even at different concentrations of the example compound and thus none of the groups showed acute cell death rates.

Therefore, it could be seen that the example compound restores the sternness and cell cycle of MSCs exposed to TNF-α/IFN-γ to quiescence.

### Experimental Example 2. Improvement of mitochondrial membrane potential and dynamics of TNF-α/IFN-γ-exposed MSCs according to treatment with example compound

### <Analysis of mitochondrial membrane potential and mitochondrial distribution>

hPD-MSCs were stained with MitoSpy Orange CMTMRos (BioLegend, SanDiego, CA, USA) to observe mitochondrial membrane potential, and stained with MitoSpy Orange CMTMRos (BioLegend, SanDiego, CA, USA) to observe mitochondrial distribution. After 72 hours with or without MOTS-c treatment, hPD-MSCs were passaged on a gelatin-coated cover glass (Sigma, St. Louis, Missouri, USA) in 12-well plates (SPL) and stained 24 hours later. The hPD-MSCs were cultured in α-MEM containing 250 nM MitoSpy Green FM and 250 nM MitoSpy Orange CMTMRo for 30 minutes. Stained hPD-MSCs were fixed with 4% paraformaldehyde (Alfa Aesar, Ward Hill, Massachusetts, USA) for 5 minutes, and then stained with 1 µg/mL Hoechst 33342^{®} (ThermoFisher) at room temperature for 5 minutes. Finally, the cover glass was mounted on a slide glass using a mounting solution (VectaShield, ThermoFisher) and samples were imaged with a confocal microscope (LSM880; Zeiss, Oberkochen, Germany). The fluorescence intensities of confocal images were quantified using ZEN 2018 Black and Blue edition software (Zeiss).

The mitochondrial membrane potential (red) and mitochondrial distribution (green) of the untreated MSC control, the TNF-α/IFN-γ-exposed MSC control (TI), and the experimental group treated with the example compound in the TNF-α/IFN-γ-exposed MSC control were confirmed using a confocal microscope by the above method, and are shown in FIG. 2A.

After exposure to TNF-α and IFN-γ, MSCs underwent metabolic conversion from quiescence to an activated state and began to age. The untreated MSC control exhibited a mitochondrial tubular network uniformly distributed around the nuclear site, whereas the TNF-α/IFN-γ exposed MSC control (TI) exhibited damaged mitochondria in a flattened and elongated form due to mitochondrial damage. It could be confirmed that, when the TNF-α/IFN-γ-exposed MSC control was treated with the example compound, the MSCs were restored to quiescence in the mitochondrial membrane potential and distribution.

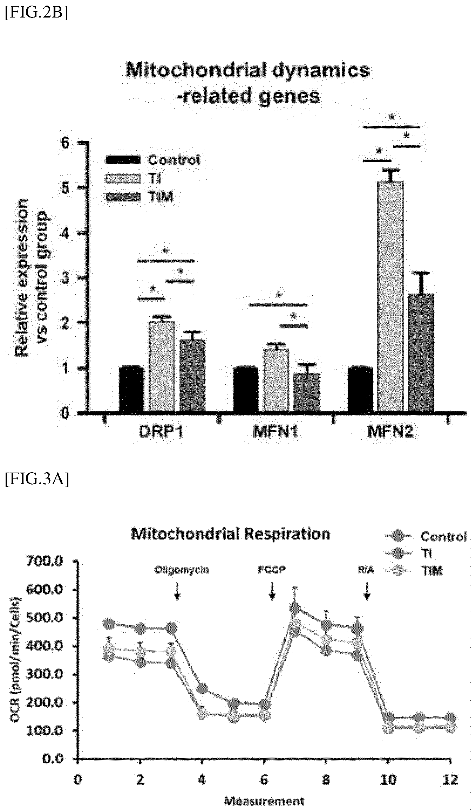

To investigate mitochondrial dynamics, real-time qPCR was performed to analyze the expression of dynamin-related protein 1 (DRP1), which is a mitochondrial division-related gene, and mitofusion 1 (MFN1) and mitofusion 2 (MFN2), which are inner mitochondrial membrane fusion-related genes. As the real-time qPCR analysis method, the same method described in Experimental Example 1-2 above was used, and the results are shown in FIG. 2B.

MFN2 was significantly affected by TNF-α and IFN-γ, and the compound of the example, TNF-α and IFN-γ exposure increased MFN2 5-fold when the untreated MSC control was compared with the TNF-α/IFN-γ exposed MSC control (TI), but when the TNF-α/IFN-γ-exposed MSC control (TI) was treated with the example compound, MFN2 expression was reduced by almost 50%. Further, it was confirmed that the expression of DRP1 and MFN1 was increased 2-fold and 1.5-fold in the TNF-α/IFN-γ-exposed MSC control (TI) compared to the untreated MSC control, but the expression levels was slightly reduced by treatment with the example compound.

Therefore, it was confirmed that, when TNF-α/IFN-γ-exposed MSCs were treated with the example compound, a mitochondrial network, which is healthy and restored to quiescence, could be formed.

### Experimental Example 3. Amelioration of oxidative stress according to example compound

### <Measurement of oxygen consumption rate (OCR)>

An oxygen consumption rate was measured to assess a mitochondrial metabolic state using a Seahorse XFp Extracellular Flux Analyzer (Agilent, Santa Clara, CA, USA). A total of 10,000 cells/well were seeded in Seahorse XFp cell culture miniplates and cultured with or without TNF-α, IFN-γ and the example compound for 24 hours prior to analysis. The growth medium was replaced with a Seahorse XF base medium containing 2 mM L-glutamine, 5.5 mM D-glucose, and 1 mM sodium pyruvate according to the composition of α-MEM. Cells were treated with 1 µM oligomycin, 0.5 µM FCCP, and 0.5 µM rotenone/antimycin A during the assay. The OCR was measured for 2 minutes after mixing for 3 minutes, and this process was repeated 12 times. A basal OCR was normalized according to the protein concentration determined by BCA analysis.

The OCRs of the untreated MSC control, the TNF-α/IFN-γ-exposed MSC control (TI), and the experimental group treated with the example compound in the TNF-α/IFN-γ-exposed MSC control were measured by the above method, and the results are shown in FIG. 3A. As shown in FIG. 3A, it could be seen that the example compound restored TNF-α/IFN-γ-exposed MSCs to quiescence.

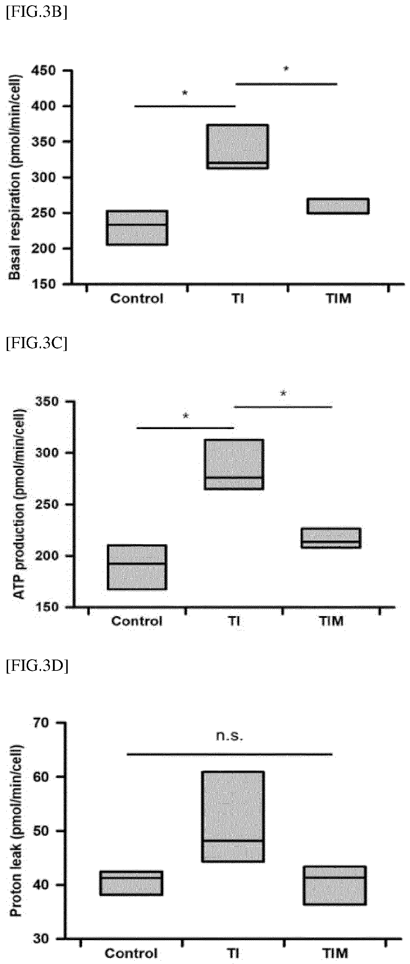

The basal respiration, ATP production amount, proton leakage, and spare respiratory capacity (SRC) for each of the above samples are shown in FIGS. 3B to 3E.

As shown in FIG. 3B, the untreated MSC control consumed about 240 pmol/min/cell, but the TNF-α/IFN-γ-exposed MSC control (TI) increased basal respiration by about 100 pmol/min/cell compared to the untreated MSC control. However, when the control group (TI) was treated with the compound of the example, the basal respiration could be decreased by about 80 pmol/min/cell.

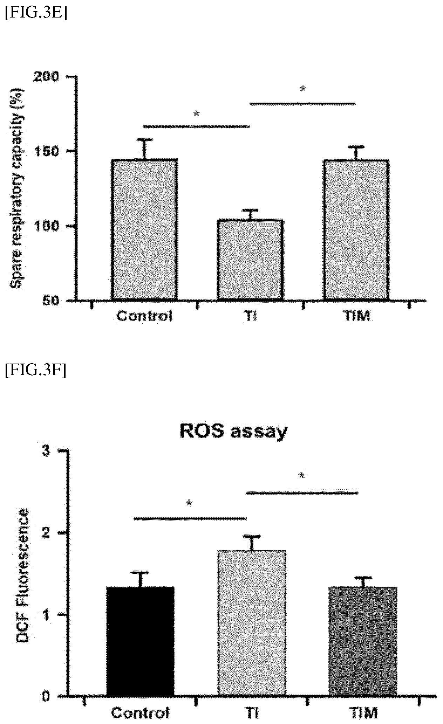

Likewise, as shown in FIG. 3C, although the oxygen consumption amount for ATP production was also higher in the TNF-α/IFN-γ-exposed MSC control (TI) by about 100 pmol/min/cell compared to the untreated MSC control, when treated with the example compound, basal respiration was downregulated by about 70 pmol/min/cell. In addition, similar trends were also shown in proton leakage according to basal respiration and ATP production (see FIG. 3D). In contrast, it was confirmed that spare respiratory capacity (SRC) was reduced by 40% in the TNF-α/IFN-γ-exposed MSC control (TI) compared to the untreated MSC control, but when the control was treated with the example compound, the amount of SRC was also restored by improving to almost the same value as the untreated MSC control (see FIG. 3E).

### <Analysis of reactive oxygen species (ROS)>

To investigate ROS levels, a total of 5 × 10³ hPD-MSCs were seeded into each well of a 96-well black-microplate (SPL, 33196) and cultured for 24 hours, and then the cells were washed and cultured in a pre-warmed Hanks' balanced salt solution (14025092, ThermoFisher) containing 50 µM H2DCFDA (D399; ThermoFisher) for 30 minutes. Fluorescence intensity at a wavelength of 555 nm was measured at a wavelength of 488 nm using a Multi-Mode MicroPlate Reader (FlexStation 3, Molecular Devices, Silicon Valley, CA, USA), and the results are shown in FIG. 3F.

As shown in FIG. 3F, although the TNF-α/IFN-γ-exposed MSC control (TI) has a 40% higher ROS value than the untreated MSC control, when the TNF-α/IFN-γ-exposed MSC control (TI) is treated with the example compound, the value can be reduced to the level of the untreated MSC control.

Thus, it was confirmed that the example compound can reduce an excessive mitochondrial oxygen consumption rate in stem cells and inhibit an increase in ROS production.

### Experimental Example 4. Improvement of aging phenotype of TNF-α/IFN-γ-exposed MSCs according to treatment with example compound

### <Measurement of SASP expression>

MSCs exposed to TNF-α/IFN-γ are known to change to pro-inflammatory phenotypes such as a senescence-associated secretory phenotype (SASP) and a damage associated molecular pattern (DAMP) and cause additional inflammation through a feedback loop.

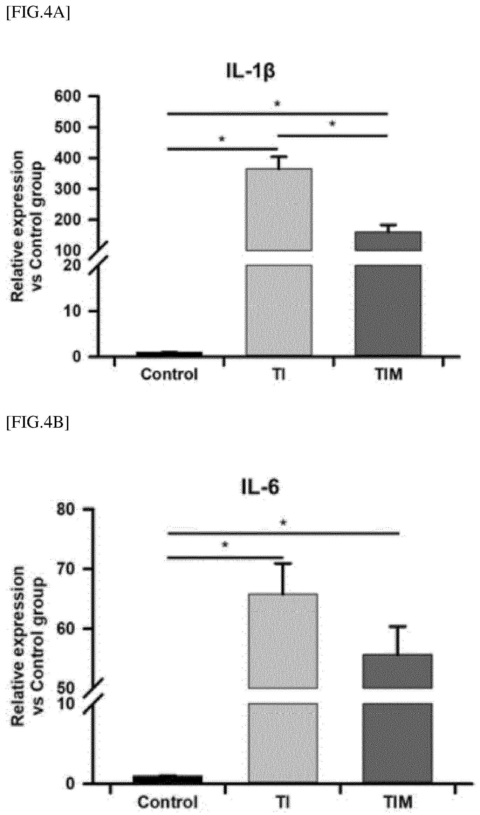

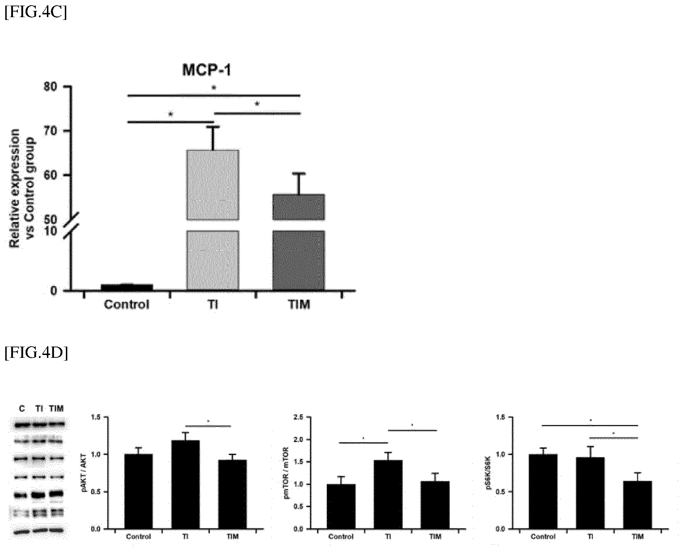

In the present experiment, the expression of IL-1β, IL-6, and monocyte chemotactic protein-1 (MCP-1), which are SASP-associated factors of the untreated MSC control, the TNF-α/IFN-γ-exposed MSC control (TI), and the experimental group treated with the example compound in the TNF-α/IFN-γ-exposed MSC control was analyzed by real-time qPCR. As the real-time qPCR analysis method, the same method described in Experimental Example 1-2 above was used, and the results are shown in FIGS. 4A to 4C.

As shown in FIGS. 4A to 4C, it was confirmed that MSCs exposed to TNF-α/IFN-γ showed a remarkable increase in the expression of IL-1β, IL-6 and MCP-1, but when the MSCs exposed to TNF-α/IFN-γ were treated with the example compound, the expression levels of all of IL-1β, IL-6, and MCP-1 decreased, and specifically IL-1β and MCP-1 were decreased by about 55% and 15%, respectively.

### <Measurement of phosphorylation-induced activation ratios of AKT/mTOR/S6K>

Further, the phosphorylation-induced activation ratios of AKT/mTOR/S6K, which represents the mTORC1 pathway, was analyzed. mTORC1 activation is known to not only induce senescence-associated phenotypes in MSCs, but also impair quiescence and sternness. In the present experiment, the phosphorylation ratio was confirmed using western blotting analysis as described below.

### -Western blotting analysis

Proteins were extracted with a Pro-Prep protein lysis buffer (iNtRON) and quantified by a protein quantitative analysis kit (BIOMAX, Seoul, Korea). Samples were then heated in 4x Laemmli sample buffer (Bio-Rad) containing 2-mercaptoethanol (Bio-Rad), and proteins were loaded onto an 8% sodium dodecyl sulfate polyacrylamide gel and electrophoresed at 60 V for 30 minutes, and then electrophoresed at 120 V for 1 hour. Thereafter, proteins were transblotted onto a nitrocellulose membrane (Bio-Rad) at 400 mA for 90 minutes, the membrane was incubated with blocking buffer (TBS-T containing 4% bovine serum albumin) for 1 hour, followed by incubation with a primary antibody solution at 4°C overnight. The primary antibodies of anti-AKT1 (ThermoFisher), anti-pAKT1 (Invitrogen, IL, USA), anti-mammalian target of rapamycin (Cell Signaling, Danvers, Massachusetts, USA), anti-pmTOR (Abeam, Cambridge, MA, USA), anti-ribosomal protein S6 kinase beta-1 (Cell Signaling), anti-pS6K (Cell Signaling), and anti-β (ThermoFisher) were used. Blotted membranes were incubated with anti-mouse (ThermoFisher) or anti-rabbit (ThermoFisher) secondary antibodies at room temperature for 1 hour.

Immunoreactive bands were detected using an enhanced chemiluminescence detection reagent (Clarity^{™} Western blot substrate, Bio-Rad) and images of the bands were obtained using ImageSaver Version 6 (ATTO, Tokyo, Japan). The intensity of each band was analyzed with CA4 analyzer software (ATTO), and each experiment was performed in triplicate under the same conditions.

The phosphorylation ratio was confirmed through the band intensities of anti-pAKT/anti-AKT, anti-pmTOR/anti-mTOR, and anti-pS6K/anti-S6K obtained by performing the above western blotting analysis, and the results are shown in FIG. 4D.

Among three molecules involved in the mTORC1 pathway, the phosphorylation ratio of mTOR was significantly upregulated by TNF-α and IFN-γ exposure, whereas AKT was scarcely affected. Although S6K phosphorylation ratios show similar values, substantially increased amounts in both S6K and PS6K were observed.

Here, when the MSC control exposed to TNF-α/IFN-γ was treated with the example compound, the phosphorylation ratios of AKT, S6K and mTOR were reduced by about 25, 40 and 30%, respectively.

Therefore, through the present experiment, it can be seen that the example compound can prevent the aging phenotype of MSCs exposed to TNF-α/IFN-γ by inhibiting the expression of SASP factors and the activation of the mTORC1 pathway, which are major causes of cellular senescence.

## Claims

1. A composition for inhibiting the inflammation or aging of stem cells, comprising, as an active ingredient, a compound of Chemical Formula 1 or a pharmaceutically acceptable salt thereof: 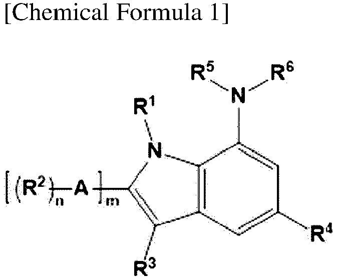 wherein,
n is an integer from 1 to 3,
m is 0 or 1,
A represents phenyl,
R¹ is hydrogen, or a C₁-C₆ alkyl,
R² represents hydrogen, a halogen or a C₁-C₆ alkoxy, or represents hydroxy-C₁₋C₆ alkyl, -(CH₂)ₚCO₂R⁷, -NHR⁸, -N(H)S(O)₂R⁷ or -NHC(O)R⁷, wherein p is an integer from 0 to 3, R⁷ represents hydrogen or a C₁-C₃ alkyl, and R⁸ represents (C₁₋C₃ alkyl)piperidinyl, or (C₁₋C₃ alkyl)sulfonyl,
R³ represents hydrogen, a halogen, a C₁-C₆ alkyl or phenyl, or -(CH₂)ₚ-heterocycle in which the heterocycle is a 5- to 6-membered ring containing one or two heteroatom(s) selected among N and O atoms, wherein p is an integer from 0 to 3, provided that R³ is phenyl when m is 0,
R⁴ represents a halogen, a C₁-C₆ alkyl, hydroxy-C₁₋C₆ alkyl, -O-phenyl, - (CH₂)ₚCO₂R⁷, -(CH₂)ₚ-heterocycle in which the heterocycle is a 5- to 6-membered ring containing one or two heteroatom(s) selected among N and O atoms, or proline-N-carbonyl, wherein p is an integer from 0 to 3, and R⁷ is as described above,
R⁵ is hydrogen, or a C₁-C₆ alkyl, and
R⁶ represents a C₁-C₆ alkyl, a C₃-C₆ cycloalkyl, a heterocycle or heterocyclyl-C₁₋C₆ alkyl, wherein the heterocycle is a 3- to 8-membered ring including 1 to 3 heteroatom(s) selected among N and O atoms, and R⁶ is optionally substituted with (C₁-C₆ alkyl)amine, hydroxy-C₁₋C₆ alkyl, or (C₁-C₆ alkyl)sulfonyl.

2. The composition of claim 1, wherein R³ represents hydrogen, a halogen, or phenyl, or represents -(CH₂)p-heterocycle in which the heterocycle is morpholino or piperazinonyl, wherein p is an integer from 0 to 1, provided that R³ is phenyl, when m is 0.
R⁴ is a halogen, a C₁-C₃ alkyl, hydroxy-C₁-C₃ alkyl, -O-phenyl, - (CH₂)ₚCO₂-ethyl, -(CH₂)ₚ-heterocycle in which the heterocycle is thiomorpholino, morpholino, piperazinonyl, or pyrrolidinyl, or proline-N-carbonyl, wherein p is an integer from 0 to 1,
R⁵ is hydrogen, or a C₁-C₃ alkyl, and
R⁶ represents a C₁-C₃ alkyl, a C₃-C₆ cycloalkyl, a heterocycle or heterocyclyl-C₁-C₃ alkyl, wherein the heterocycle is tetrahydro-2H-pyran, or piperidinyl, and when R⁶ is a heterocycle or heterocyclyl-C₁-C₃ alkyl, R⁶ is optionally substituted with (C₁-C₆ alkyl)amine, hydroxy-C₁₋C₆ alkyl, or (C₁-C₆ alkyl)sulfonyl.

3. The composition of claim 1, wherein the compound of Chemical Formula 1 is any one selected from the following compound group.
<1> 5-[(1,1-dioxido-4-thiomorpholinyl)methyl]-2-phenyl-N-(tetrahydro-2H-pyran-4-yl)-1H-indole-7-amine; <2> ethyl 7-(cyclopentylamino)-2-phenyl-1H-indole-5-carboxylate; <3> (7-(cyclopentylamino)-2-phenyl-1H-indol-5-yl)methanol; <4> 5-chloro-N,1-dimethyl-2-phenyl-N-(tetrahydro-2H-pyran-4-yl)-1H-indole-7-amine; <5> 4-((7-(cyclopentylamino)-2-(3-fluorophenyl)-1H-indol-5-yl)methyl)piperazine-2-one; <6> 4-((2-phenyl-7-(((tetrahydro-2H-pyran-4-yl)methyl)amino)-1H-indol-5-yl)methyl)thiomorpholine 1,1-dioxide; <7> 5-chloro-N-(1-methylpiperidin-4-yl)-2-phenyl-1H-indole-7-amine; <8> 5-phenoxy-2-phenyl-N-(tetrahydro-2H-pyran-4-yl)-1H-indole-7-amine; <9> 5-chloro-3-(morpholinomethyl)-2-phenyl-N-(tetrahydro-2H-pyran-4-yl)-1H-indole-7-amine; <10> 2-(4-((5-fluoro-2-phenyl-1H-indol-7-yl)amino)piperidin-1-yl)ethan-1-ol; <11> N-(4-(5-chloro-7-(cyclopentylamino)-1H-indol-2-yl)phenyl)methanesulfonamide; <12> 5-chloro-3-phenyl-N-(tetrahydro-2H-pyran-4-yl)-1H-indole-7-amine; <13> 4-((2-(3-fluorophenyl)-7-((tetrahydro-2H-pyran-4-yl)amino)-1H-indol-5-yl)methyl)thiomorphline 1,1-dioxide; <14> 5-chloro-N-cyclopentyl-2-(4-((1-methylpiperidin-4-yl)amino)phenyl)-1H-indole-7-amine; <15> 4-((7-(isopentylamino)-2-(4-methoxyphenyl)-1H-indol-5-yl)methyl)thiomorpholine 1,1-dioxide; <16> N-(4-(7-(cyclopentylamino)-5-((1,1-dioxidothiomorpholino)methyl)-1H-indol-2-yl)phenyl)acetamide; <17> 4-((3-bromo-2-phenyl-7-((tetrahydro-2H-pyran-4-yl)amino)-1H-indol-5-yl)methyl)thiomorpholine 1,1-dioxide; <18> 4-((5-chloro-2-phenyl-7-((tetrahydro-2H-pyran-4-yl)amino)-1H-indol-3-yl)methyl)piperazine-2-one; <19> 4-((7-(methyl(tetrahydro-2H-pyran-4-yl)amino)-2-phenyl-1H-indol-5-yl)methyl)thiomorpholine 1,1-dioxide; <20> 5-methyl-N-(1-(methylsulfonyl)piperidin-4-yl)-2-phenyl-1H-indole-7-amine; <21> N-(4-(5-(1,1-dioxidothiomorpholino)-7-((tetrahydro-2H-pyran-4-yl)amino)-1H-indol-2-yl)phenyl)acetamide; <22> 4-((7-((1-(methylsulfonyl)piperidin-4-yl)amino)-2-phenyl-1H-indole-5-yl)methyl)thiomorpholine 1,1-dioxide; <23> N1-(5-chloro-2-phenyl-1H-indol-7-yl)-N4-methylcyclohexane-1,4-diamine; <24> methyl 2-(3-(5-chloro-7-((tetrahydro-2H-pyran-4-yl)amino)-1H-indol-2-yl)phenyl)acetate; <25> (2-phenyl-7-((tetrahydro-2H-pyran-4-yl)amino)-1H-indol-5-carbonyl)-D-proline; <26> (3-(5-chloro-7-((tetrahydro-2H-pyran-4-yl)amino)-1H-indol-2-yl)phenyl)methanol; <27> N-cyclopentyl-2-phenyl-5-(2-(pyrrolidin-1-yl)ethyl)-1H-indole-7-amine; <28> methyl 2-(4-(5-chloro-7-((tetrahydro-2H-pyran-4-yl)amino)-1H-indol-2-yl)phenyl)acetate; <29> methyl 4-(5-chloro-7-(cyclopentylamino)-1H-indol-2-yl)benzoate; <30> 2-(4-(5-chloro-7-((tetrahydro-2H-pyran-4-yl)amino)-1H-indol-2-yl)phenyl)ethan-1-ol; <31> 3-bromo-5-(morpholinomethyl)-2-phenyl-N-(tetrahydro-2H-pyran-4-yl)-1H-indole-7-amine; and <32> 4-((3-phenyl-7-((tetrahydro-2H-pyran-4-yl)amino)-1H-indol-5-yl)methyl)thiomorpholine 1,1-dioxide.

4. The composition of claim 1, wherein the compound of Chemical Formula 1 is a compound of the following Chemical Formula 2.

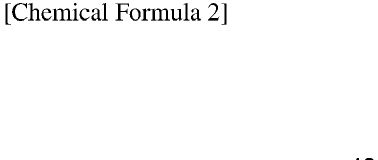

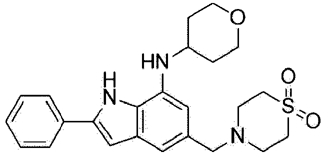

5. The composition of claim 1, wherein the stem cells are mesenchymal stem cells derived from the human placenta.

6. The composition of claim 1, wherein the stem cells are *in vivo* or *ex vivo* stem cells.

7. The composition of claim 1, wherein the inhibition of inflammation or aging of stem cells restores stem cells damaged by exposure to inflammatory cytokines to normal levels.

8. The composition of claim 7, wherein the inflammatory cytokine is TNF-α and/or IFN-γ.

9. The composition of claim 1, wherein the compound of Chemical Formula 1 or the pharmaceutically acceptable salt thereof in the composition is comprised at a concentration of 0.05 µM to 10 µM.

10. The composition of claim 1, wherein the inhibition of inflammation or aging of stem cells has one or more characteristics selected from the group consisting of restoration of quiescence of stem cells or improvement of homeostasis; cell cycle regulation or maintenance of sternness; and stem cell phenotype restoration.

11. A method for inhibiting the inflammation or aging of stem cells, the method comprising: treating stem cells with a compound of Chemical Formula 1 or a pharmaceutically acceptable salt thereof: 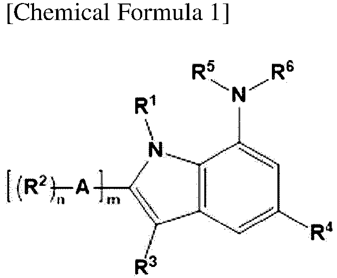 in the formula, R¹ to R⁶, A, n and m are each the same as defined in claim 1.

12. A method for preparing inflammation- or aging-inhibited stem cells, the method comprising: treating stem cells with a compound of Chemical Formula 1: 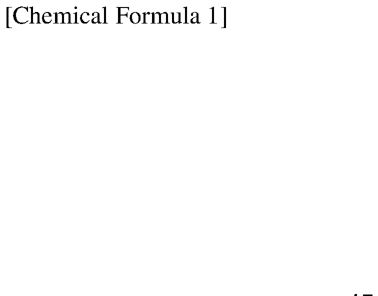 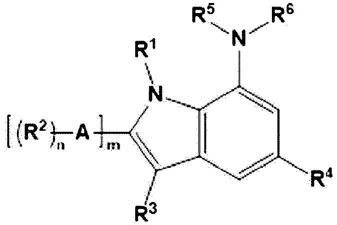 in the formula, R¹ to R⁶, A, n and m are each the same as defined in claim 1.

13. Stem cells prepared by the method of claim 12.

14. A pharmaceutical composition for preventing or treating an inflammatory disease, comprising the stem cells of claim 13 as an active ingredient.

15. The composition of claim 14, wherein the inflammatory disease is selected from the group consisting of dermatitis, allergies, atopy, asthma, conjunctivitis, periodontitis, rhinitis, tympanitis, pharyngolaryngitis, tonsillitis, pneumonia, gastric ulcer, gastritis, Crohn's disease, colitis, peritonitis, osteomyelitis, phlegmon, cerebromeningitis, encephalitis, pancreatitis, stroke, acute bronchitis, chronic bronchitis, hemorrhoids, gout, ankylosing spondylitis, rheumatic fever, systemic lupus erythematosus, fibromyalgia, psoriatic arthritis, osteoarthritis, rheumatoid arthritis, infectious arthritis, periarthritis, tendinitis, tenosynovitis, peritendinitis, myositis, hepatitis, cystitis, nephritis, Sjogren's syndrome, multiple sclerosis, inflammatory bowel disease, transplantation rejection, bronchopulmonary dysplasia (BPD), chronic obstructive pulmonary disease (COPD) and acute and chronic inflammatory diseases.
